# EUROPEAN PATENT SPECIFICATION

(11) **EP 2 088 426 B1**
(45) Date of publication and mention of the grant of the patent: **15.05.2013**
(21) Application number: 07829381.8
(22) Date of filing: 02.10.2007
(51) Int. Cl.: G01N 27/62, G01N 33/48, G01N 33/483

(54) **MASS SPECTROMETRY OF BIOLOGICAL SAMPLE USING IMMUNOPRECIPITATION**
MASSENSPEKTROMETRIE EINER BIOLOGISCHEN PROBE UNTER VERWENDUNG VON IMMUNPRÄZIPITATION
SPECTROMÉTRIE DE MASSE D'UN ÉCHANTILLON BIOLOGIQUE PAR IMMUNOPRÉCIPITATION

(30) Priority: 29.11.2006 JP 2006322646
(43) Date of publication of application: 12.08.2009
(73) Proprietor: Shimadzu Corporation, Kyoto-shi Kyoto 604-8511 (JP)
(72) Inventor: SHIMADA, Takashi, Kyoto-shi Kyoto 604-8511 (JP); TOYAMA, Atsuhiko, Kyoto-shi Kyoto 604-8511 (JP); SATO, Taka-Aki, Kyoto-shi Kyoto 604-8511 (JP)
(74) Representative: Kilian Kilian & Partner
(86) International application number: PCT/JP2007/069643
(87) International publication number: WO 2008/065806

(56) References cited:
- JP-A- 2006 284 389
- MILES RODNEY R ET AL: "Analysis of BCL6-interacting proteins by tandem mass spectrometry" MOLECULAR & CELLULAR PROTEOMICS, vol. 4, no. 12, December 2005 (2005-12), pages 1898-1909, XP002550740 ISSN: 1535-9476
- GRONBORG M ET AL: "A Mass Spectrometry-based Proteomic Approach for Identification of Serine/Threonine-phosphorylated Proteins by Enrichment with Phospho-specific Antibodies" MOLECULAR & CELLULAR PROTEOMICS, AMERICAN SOCIETY FOR BIOCHEMISTRY AND MOLECULAR BIOLOGY, INC, US, vol. 1, 1 July 2002 (2002-07-01), pages 517-527, XP003008384 ISSN: 1535-9476
- PORTELIUS ERIK ET AL: "Determination of beta-amyloid peptide signatures in cerebrospinal fluid using immunoprecipitation-mass spectrometry" JOURNAL OF PROTEOME RESEARCH, vol. 5, no. 4, April 2006 (2006-04), pages 1010-1016, XP002550741 ISSN: 1535-3893
- KIM S.-A. ET AL.: 'Regulation of myotubularin-related (MTMR) 2-phosphatidylinositol phosphatase by MTMR5, a catalytically inactive phosphatase' PROC. NATL. ACAD. SCI. USA vol. 100, no. 8, 15 April 2003, pages 4492 - 4497, XP003022908
- GOBOM J. ET AL.: 'Detection and Quantification of Neurotensin in Human Brain Tissue by Matrix-Assisted Laser Desorption/Ionization Time-of-Flight Mass Spectrometry' ANAL. CHEM. vol. 72, no. 14, 15 July 2000, pages 3320 - 3326, XP002982368
- SURESH BABU C.V. ET AL.: 'Analysis of substance P in rat brain by means of immunoaffinity capture and matrix-assisted laser desorption/ionization time-of-flight mass-spectrometry' J. CHROMATOGR. vol. 807, no. 2, 25 May 2004, pages 307 - 313, XP004518846
- SEEBER S. ET AL.: 'Formation of Molecular Complexes by N-Methyl-D-aspartate Receptor Subunit NR2b and Ryanodine Receptor 2 in Neonatal Rat Myocard' J. BIOL. CHEM. vol. 279, no. 20, 14 May 2004, pages 21062 - 21068, XP003022909

## Description

### TECHNICAL FIELD

The present invention relates to the fields of medicine, applied chemistry, basic biology, structural biology, clinical diagnosis and the like. Concretely, the present invention relates to a method for mass spectrometry of biological sample using immunoprecipitation. In particular, the present invention relates to a mass spectrometry intended for detection of a biomarker molecule from a clinical sample.

### BACKGROUND ART

Ciphergen Biosystems Inc. has developed such a method that a functional group such as antibody is immobilized on a chip surface, and a specific molecule is captured by a functional group specific binding, and after washing and removing undesired components with water or a buffer, laser is applied and the captured specific molecule is analyzed by mass spectrometry (SELDI protein chip (R) system) (see WO 1996/036732 pamphlet (Patent document 1)). Since not all the molecules can be practically captured by one chip, several kinds of chips such as a hydrophobic chip, an ion exchange chip, and a metal ion affinity chip are used and individually added with the same sample, and expression is analyzed for each chip.

On the other hand, Journal of Neurochemistry, 2003, 84, 602-609 (Non-patent document 1) describes that amyloid β is caused to immunoprecipitate using Protein G-sepharose, the obtained antibody-sepharose slurry is washed with PBS and HEPES, and the washed antibody-sepharose slurry is directly applied onto MALDI target plate, and measurement is conducted.

Further, on the other hand, N. Leigh Anderson and Norman G. Anderson, Mol. Cell. Proteomics, Nov 2002; 1: 845-867 (Non-patent document 2) describes that a significant symptom is detected even with a slight amount of tissue of a leakage protein product from tissue, and that the blood level thereof is ng/mL order as a result of a spike test.

Also, great importance of the abundance ratio of amyloid peptides, particularly (1-40)/(1-42) ratio in clinical diagnosis, is suggested, for example, in NATURE NEUROSCIENCE 7(9), 954-960, 2004 (Non-patent document 3), HUMAN MUTATION 27 (7), 686-695, 2006 (Non-patent document 4), Dement Geriatr Cogn Disord 2007;23: 316-320 (Non-patent document 5), and the like.

Concretely, the above Non-patent document 3 describes that Aβ targets on the vascular structure of a mouse model having intracranial hemorrhage with hereditary amyloidosis, the above Non-patent document 4 describes that the average age of onset of familial Alzheimer's disease by presenilin mutation correlates with both increase in Ab42 and decrease in Ab40, and the above Non-patent document 5 describes prediction of Alzheimer's disease using the A42/A40 ratio of cerebrospinal fluid in a patient having mild cognitive impairment.
Patent document 1: WO 1996/036732 pamphlet
Non-patent document 1: "Journal of Neurochemistry", 2003, 84, p.602-609
Non-patent document 2: N. Leigh Anderson and Norman G. Anderson, "Molecular & Cellular Proteomics" Nov 2002; 1, p.845-867
Non-patent document 3: "NATURE NEUROSCIENCE", 2004, 7(9), p.954-960
Non-patent document 4: "HUMAN MUTATION", 2006, 27(7), p.686-695
Non-patent document 5: "Dementia and Geriatric Cognitive Disorder", 2007, 23, p.316-320

JP 2006 284389 A discloses a method for evaluating and discriminating the normal state and the abnormal state of a living body using a system of xenodiagnosis based on protein, partial protein and/or partial peptide or profiles of them. Said method may comprise an immunoprecipitation and detection method using an antibody affinity gel. Prior to the mass analysis by MALDI-TOF, the peptide to be analyzed is separated from the antibody affinity gel and thus from the Immuncomplex and the recovered peptide is subjected to mass spectrometry.

Miles Rodney R., et al., Molecular & Cellular Proteomics, vol. 4, no. 12, December 2005, p. 1898-1909, describe the analysis of BCL-interacting proteins by tandem mass spectrometry, wherein proteins are isolated by co-immunoprecipitation with an anti-BLC6 antibody and identified using MS/MS. The antibody-selected proteins are eluted from the agarose beads, resolved by SDS-PAGE, digested and extracted from the gel before they are analyzed by LC-MS/MS.

Grønborg M., et al., Molecular & Cellular Proteomics, vol. 1, no. 7, July 2002, p. 517-527, Kim S.-A., et al., Proc. Natl. Acad. Scl. USA, vol. 100, no. 8, April 2003, p. 4492-4497, and Seeber S., et al., J. Biol. Chem., vol. 279, no. 20, May 2004, p. 21062-21068, describe immunoprecipitation methods comprising separation of proteins from an immunoprecipitate, resolution by SDS/PAGE, digestion with trypsin and extraction from the gel prior to the MALDI-TOF analysis of the peptides.

Portelius Erik, et al., Journal of Proteome Research, vol. 5, no. 4, April 2006, p. 1010-1016, Gobom J., et al., Anal. Chem., vol. 72, no. 14, July 2000, p. 3320-3326, and Suresh Babu C. V., et al., J. Chromatogr., vol. 807, no. 2, May 2004, p. 307-313 all describe methods based on immunoprecipitation using beads combined with MALDI-TOF-MS. In said methods, the peptides bound to the beads are always separated from the beads, and thus from the immunoprecipitate, in an elution step prior to the MALDI-TOF-MS analysis.

### DISCLOSURE OF THE INVENTION

### Object of the Invention

In the SELDI method, there is no critical means for identifying a molecule captured by a functional group. In brief, when a molecule fluctuates in the SELDI method (for example, when a molecule captured by an ion exchange chip fluctuates), it is impossible to examine the cause of the fluctuation. That is, the fundamental cause of the fluctuation, in particular, the form of the molecule cannot be examined because the method fails to identify the molecule while it is able to follow the fluctuation in the molecule.

In the method disclosed in Journal of Neurochemistry, 2003, 84, 602-609, washing of antibody-sepharose obtained by immunoprecipitation is conducted with the use of a normal buffer. However, the washing effect by using the normal buffer is insufficient for the purpose of detecting an objective molecule by mass spectrometry. Therefore, the detection sensitivity in the later mass spectrometry is low.

As described above, great importance of the abundance ratio of amyloid peptides, in particular (1-40)/(1-42) ratio, is suggested in several documents. The detection of amyloid (1-40) and amyloid (1-42) can be realized by commonly used ELISA method with identical antibody. However, the ELISA method fails to distinguish them from each other. In brief, when the ELISA method is used, distinguishing amyloid (1-40) and amyloid (1-42) from each other requires a specific antibody for each of them.

It is an object of the present invention to provide an analysis method for a biological sample intended for detecting a clinically significant molecule, or a fragment derived therefrom by splicing or the like.

Also, in the present invention, we focused on screening of biological molecules to be detected, as one purpose of fundamental solution of the SELDI method. In the present invention, we also focused on conducting washing operation strictly in washing of immunoprecipitate used in a conventional immunoprecipitation technique. Therefore, it is an object of the present invention to provide an analysis method for a biological sample with clinical significance, which is a system being simple to such an extent that it can be applied to a clinical field, and capable of capping a molecule ionization suppression in mass spectrometry.

It is an object of the present invention to provide an analysis method for a biological sample that is usable in clinical cases by making a simple experimental system, and has wide application range for various biomarker tests responding to diseases.

### Summary of the Invention

As a result of diligent effort, the present inventors have found that the above object of the present invention is achieved by concentrating an objective biological molecule to be detected by immunoprecipitation, and washing the immunoprecipitate with a washing liquid that assists mass spectrometry, and have accomplished the present invention.

The present invention includes the followings.
(1) Mass spectrometry of a biological sample using immunoprecipitation, as defined in claim 1 comprising the steps of:
   preparing a sample containing an objective biological molecule selected from a peptide and a protein;
   concentrating the biological molecule in the sample by immunoprecipitation using an antibody-bound carrier or a carrier to which a molecule capable of specifically binding to an antibody is bound;
   washing an immunoprecipitate of the biological molecule by using an aqueous solution containing a charge neutralizing agent as a washing liquid; and
   detecting the biological molecule by subjecting the washed immunoprecipitate directly to mass spectrometry.
(2) The mass spectrometry of a biological sample using immunoprecipitation according to (1), wherein the sample containing an objective biological molecule is contained in a body fluid selected from the group consisting of blood, urine, cerebrospinal fluid and body secretion fluid.
(3) The mass spectrometry of a biological sample using immunoprecipitation according to (1) or (2), wherein the sample containing an objective biological molecule is a clinical sample.
(4) The mass spectrometry of a biological sample using immunoprecipitation according to any one of (1) to (3), wherein the biological molecule is selected from the group consisting of a biomarker, biological molecule of a biomarker candidate, and a fragment derived from the biomarker or the biological molecule of a biomarker candidate by splicing.
(5) The mass spectrometry of a biological sample using immunoprecipitation according to (4), wherein the biomarker and the biological molecule of a biomarker candidate are responsive to a disease selected from the group consisting of a cancer, a brain disease, an immune disease, a liver disease, a renal disease and an eye disease.

The following (6) relates to a carrier for immunoprecipitation used in the concentrating step. (6) The mass spectrometry of a biological sample using immunoprecipitation according to any one of (1) to (5), wherein the carrier is selected from the group consisting of agarose, sepharose, polyacrylamide, polyethylene glycol, polystyrene, polyethylene, polypropylene, polyester, polyacrylonitrile, (meth)acrylate ester polymer, fluorine resin, metal complex resin, glass, metal and magnetic substance.

The following (7) relates to a charge neutralizing agent used in the washing step. As an effect of using the charge neutralizing agent, the capability of capping a molecule ionization suppression in mass spectrometry may be recited. In the washing step, it keeps an appropriate pH suited for maintaining the form of the immunoprecipitate generated in the concentrating step (that is, maintaining the condition in which release of the objective biological molecule due to weakening of the antigen-antibody binding force in the immunoprecipitate does not occur).
(7) The mass spectrometry of a biological sample using immunoprecipitation according to any one of (1) to (6), wherein the aqueous solution containing the charge neutralizing agent is selected from the group consisting of ammonia water and aqueous solution of ammonium salt.

In addition to the capability of neutralizing the charges and keeping an appropriate pH, as an effect of using the charge neutralizing agent described above, it may be recited that it would not interfere with mass spectrometry as background when used in an appropriate concentration.

The following (8) and (9) relate to the mass spectrometry step.
(8) The mass spectrometry of a biological sample using immunoprecipitation according to any one of (1) to (7), wherein in the mass spectrometry, multi-stage tandem mass spectrometry is conducted.

By conducting multi-stage tandem mass spectrometry, it is possible to identify the biological molecule, and to know splicing information and post-translational modification information.
(9) The mass spectrometry of a biological sample using immunoprecipitation according to any one of (1) to (8), wherein in the mass spectrometry, the biological molecule is quantified by measuring the washed immunoprecipitate in the presence of an internal standard substance.

The following disclosure (10) and (11) relate to use of a surfactant.
(10) The mass spectrometry of a biological sample using immunoprecipitation according to the above, further comprising, prior to the mass spectrometry step, containing a nonionic surfactant in the sample containing the biological molecule or in the washing liquid for washing the immunoprecipitate of the biological molecule.
(11) Mass spectrometry of a biological sample using the immunoprecipitation, wherein the surfactant is a nonionic surfactant.

Preferably, the surfactant has a sugar chain as a hydrophilic group.

Preferably, the surfactant has a linear alkyl chain having 8 to 12 carbon number as a surface-active group.

By using the surfactant in the methods of the above (10) and (11), for example, it is possible to reduce nonspecifically adsorbed molecules to the immunoprecipitate and to make the condition of the immunoprecipitation mild by uniformity and stability of the solution and efficient collection of the objective substance, and the like.

The following disclosure (12) relates to analysis of a mixed sample of a plurality of biological molecules.
(12) Mass spectrometry of a biological sample using the immunoprecipitation, wherein the biological molecule includes a plurality of different biological molecules capable of binding to an identical antibody; in the concentrating step, the antibody is one kind of antibody; in the mass spectrometry step, the plurality of different biological molecules are detected distinguishedly.

By the method of the above (12), it is possible to find a mixing ratio of proteins in a trace amount which is very important in clinical diagnosis by a very simple method in a concurrent analysis of the identical sample.

According to the present invention, it is possible to conduct analysis for a biological sample intended for detecting a molecule with clinical significance and a fragment derived therefrom by splicing or the like. According to the present invention, it is possible to conduct an analysis for a biological sample with clinical significance, which is a system being simple to such an extent that it can be applied to a clinical field, and capable of capping a molecule inhibiting ionization in mass spectrometry. According to the present invention, it is possible to conduct an analysis for a biological sample that is usable in clinical cases by making a simple experimental system and has wide application range for various biomarker tests responding to diseases.

Concretely, the following effects are obtained by the present invention for example when compared with the conventional SELDI method. For example, in contrast to the SELDI method which requires a special SELDI chip, the method of the present invention can use general IP beads and the like, and is excellent in versatility. Also, in contrast to the SELDI method which requires a SELDI scanner for analysis, the method of the present invention makes it possible to conduct the analysis by all MALDI-based mass spectrometries, and is excellent in versatility. Further, in contrast to the SELDI method which requires SELDI peripheral devices, the method of the present invention is simple in procedures and is excellent in versatility because its experimental system is constructed based on the immunoprecipitation method that can be conducted in various basic and clinical laboratories.

For example, in comparison with methods such as the conventional ELISA, enzyme immunoassay and the like using an antibody, the method of the present invention can detect a molecule in a level such that it is undetectable by such conventional methods, and is excellent in terms of sensitivity. Concretely, even when the concentration of the objective biological molecule is a low level of several ng/ml, for example, 1 to 5 ng/ml order, the detection is possible. This order corresponds to blood level of the biological molecule at the time when a symptom such as a specific disease starts developing by the biological molecule. Therefore, the present invention can be said to be a method having such sensitivity that is sufficiently bearable to the concentration required as that of a biomarker.

Further, in the method of the present invention, since the objective biological molecule is measured as an immunoprecipitate, it is possible to detect a molecule that immunoprecipitates together with the objective biological molecule. That is, it is also possible to examine interactions with the objective biological molecule.

The method of the present invention also makes it possible to detect a peptide mixture in a trace amount scale as an ion peak having mass difference by mass spectrometry, so that a mixing ratio of proteins in a trace amount which is very important in clinical diagnosis, such as amyloid (1-40)/(1-42) ratio, can be found by a very simple method.

### BRIEF DESCRIPTION OF THE DRAWINGS

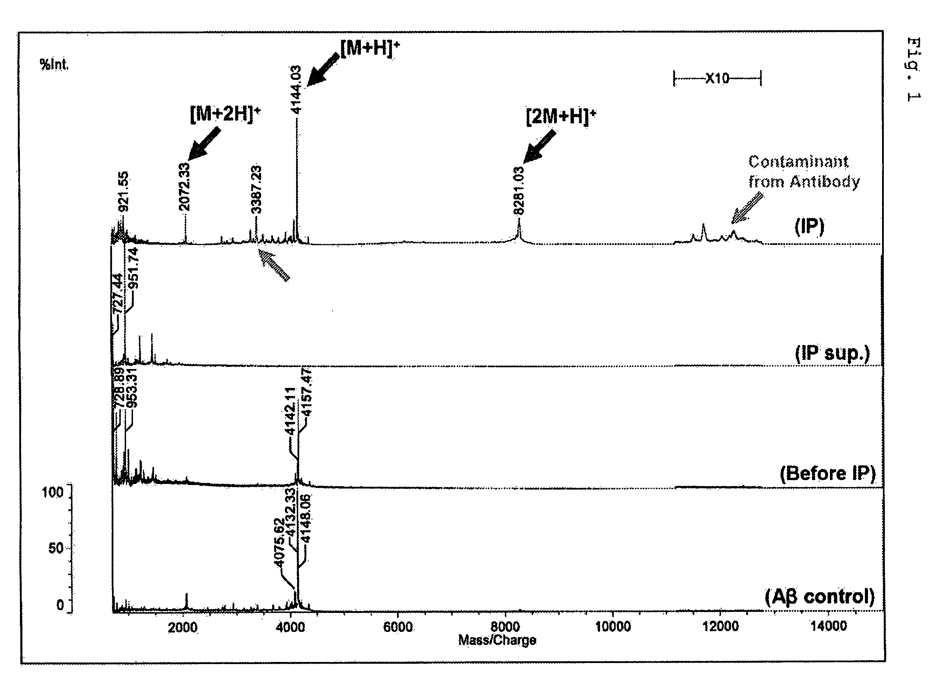

Fig. 1 shows, in Example 1, mass spectrum (IP) from an immunoprecipitate obtained by conducting IP-MS on a sample containing 10 pmol amyloid peptide in a buffer, together with mass spectrum (Aβ) from amyloid (1-38) control, mass spectrum (Before IP) from a solution just after mixing with an antibody, and mass spectrum (IP sup.) from the supernatant after immunoprecipitation.

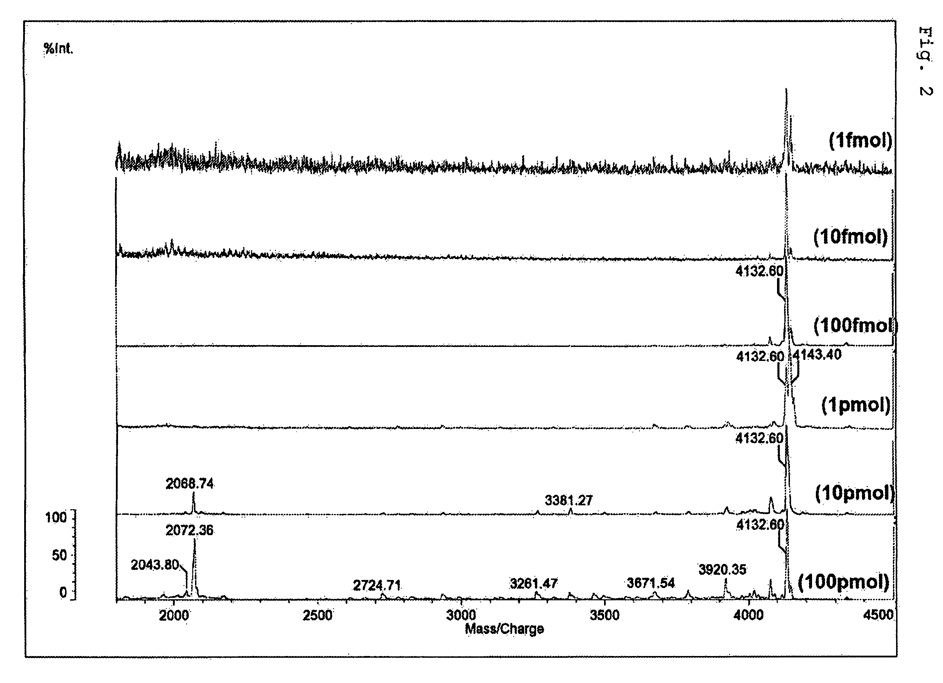

Fig. 2 shows, in Example 2, mass spectrums obtained by conducting IP-MS on samples respectively containing 100 pmol, 1 pmol, 100 fmol, 10 fmol, and 1 fmol amyloid peptide in a buffer, together with the mass spectrum (amount of amyloid peptide (1-38) in sample: 10 pmol) obtained in Example 1.

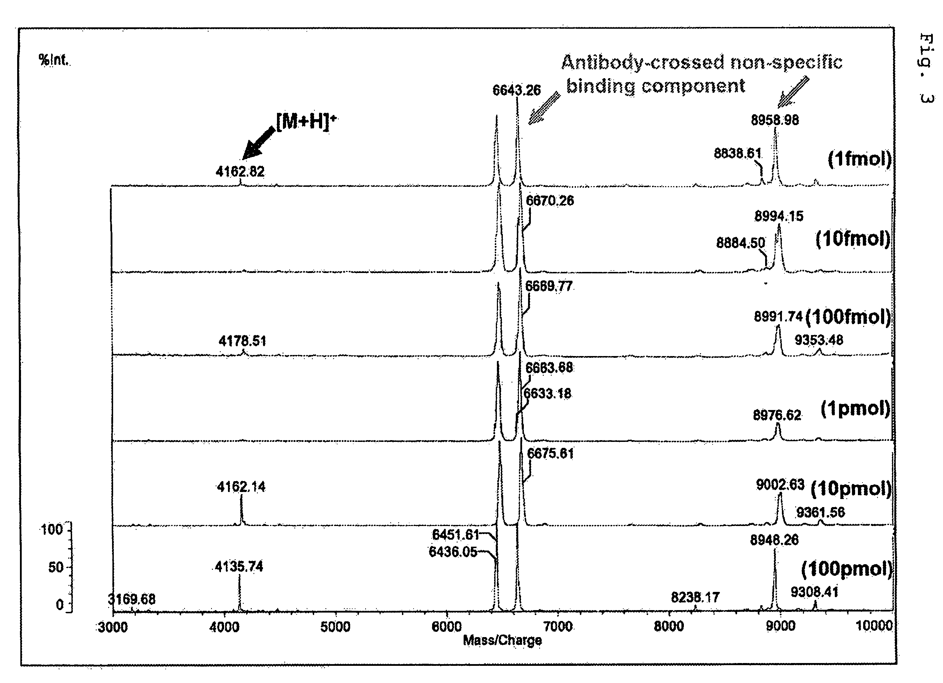

Fig. 3 shows, in Example 3, mass spectrums obtained by conducting IP-MS on samples respectively containing 100 pmol, 10 pmol, 1 pmol, 100 fmol, 10 fmol, and 1 fmol amyloid peptide in serum.

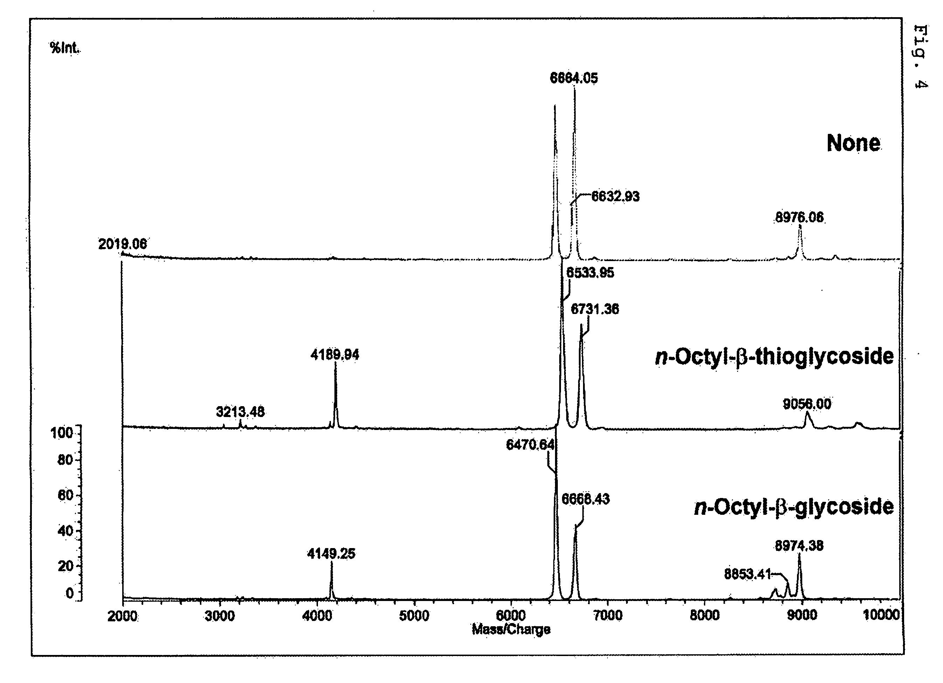

Fig. 4 shows, in Example 4, mass spectrums obtained by conducting IP-MS on samples containing 1 pmol amyloid peptide in serum added with surfactants (n-Octyl-β-D-thioglycoside and n-Octyl-β-D-glycoside), respectively, together with mass spectrum (None) obtained from a control not added with any surfactant.

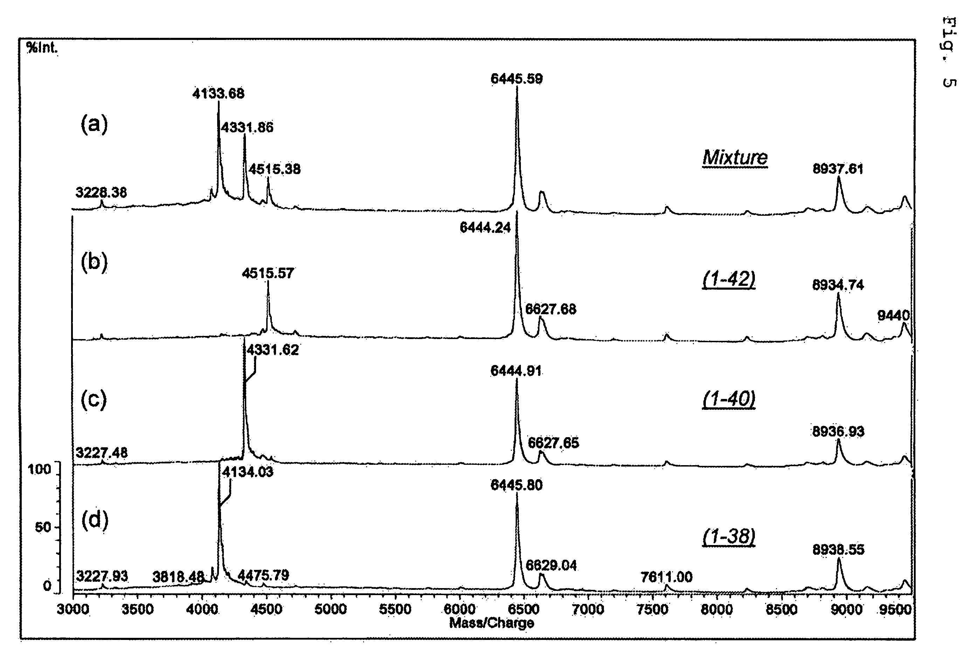

Fig. 5 shows, in Example 5, (a) mass spectrum of an amyloid peptide mixture (mixture of 500 fmol amyloid peptide (1-42), 500 fmol amyloid peptide (1-40) and 500 fmol amyloid peptide (1-40)) (Mixture), together with (b) mass spectrum of 500 fmol amyloid peptide(1-42), (c) mass spectrum of 500 fmol amyloid peptide(1-40), and (d) mass spectrum of 500 fmol amyloid peptide(1-38).

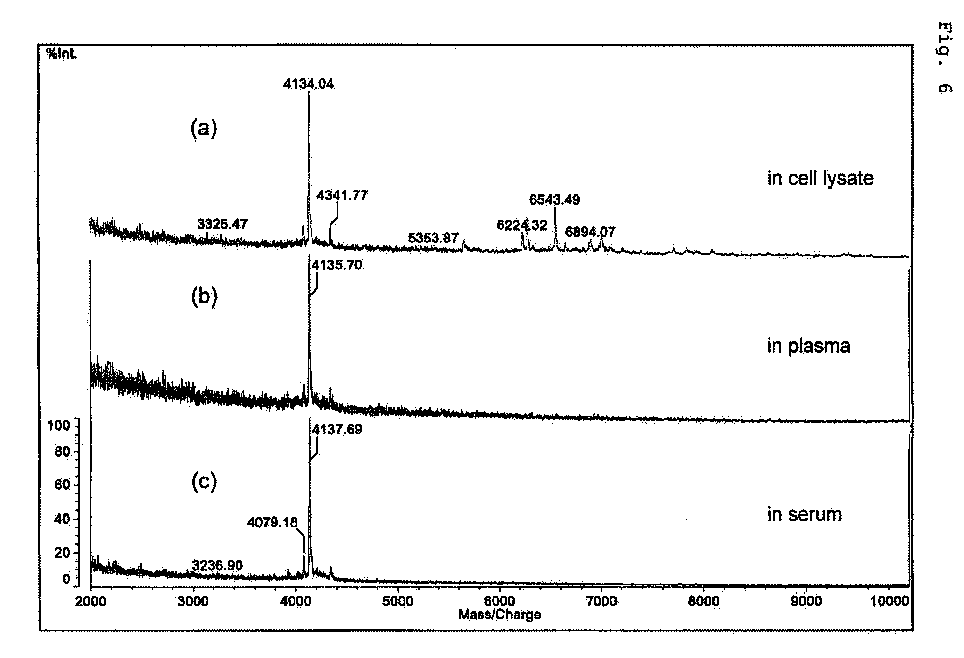

Fig. 6 shows, in Example 6, (a) mass spectrum of 500 fmol amyloid in cell lysate, (b) mass spectrum of 500 fmol amyloid in plasma, and (c) mass spectrum of 500 fmol amyloid in serum.

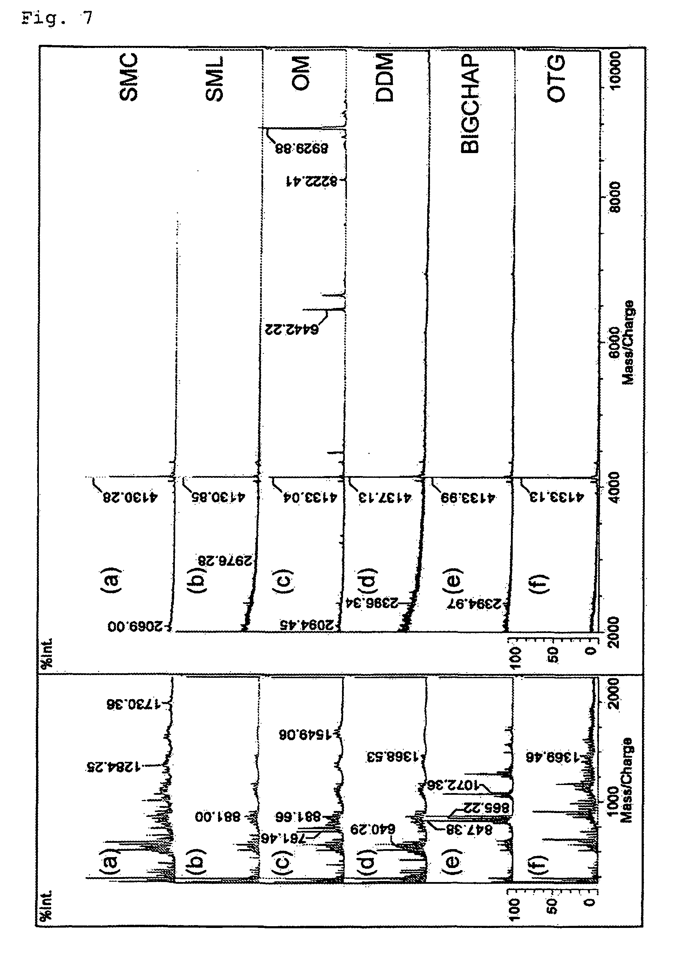

Fig. 7 shows, in Example 7, mass spectrums obtained by conducting IP-MS on samples containing 1 pmol amyloid peptide in serum added with surfactants ((a): sucrose monocaprate (SMC), (b): sucrose monolaurate (SML), (c): n-octyl-β-D-maltoside (OM), (d): n-dodecyl-β-D-maltoside (DDM), (e): deoxy BIGCHAP (BIGCHAP), and (f): n-octyl-β-D-thioglycoside (OTG)), respectively.

### MODES FOR CARRYING OUT THE INVENTION

The mass spectrometry of a biological sample using immunoprecipitation in the present invention comprises:
step of preparing a sample containing an objective biological molecule selected from a peptide and a protein;
step of concentrating the biological molecule in the sample by immunoprecipitation using an antibody-bound carrier or a carrier to which a molecule capable of specifically binding to an antibody is bound;
step of washing an immunoprecipitate of the biological molecule by using an aqueous solution containing a charge neutralizing agent as a washing liquid; and
step of detecting the biological molecule by directly subjecting the washed immunoprecipitate to mass spectrometry.

### [1. Step of preparing a sample containing an objective biological molecule]

In the present invention, a sample containing an objective biological molecule is usually a body fluid containing the objective biological molecule. The body fluid include, but are not limited to, blood, urine, cerebrospinal fluid, bodily secretion liquid and the like. As the body fluid, treated fluids, culture fluids and the like that are obtained by conducting a pretreatment such as partial purification or culture appropriately by a person skilled in the art may also be accepted. As the blood, whole blood, plasma, and serum may be recited. The present invention is particularly useful when the sample containing an objective biological molecule is a clinical sample.

As the biological molecule, a biomarker, a biological molecule of a biomarker candidate, and a fragment derived therefrom by splicing or the like may be recited. The biomarker and the biological molecule of a biomarker candidate are not particularly limited, and wide and various biomarkers may be a subject to be detected in the present invention. For example, biological molecules that respond to diseases such as cancers, brain diseases (e.g., Alzheimer), immune diseases, liver diseases (e.g., hepatic cirrhosis), renal diseases, eye diseases and the like may be recited. The biological molecule is mainly a protein.

### [2. Step of concentrating an objective biological molecule]

In this step, the objective biological molecule is precipitated by immunochemical technique for the purpose of concentrating the biological molecule. Any method is acceptable insofar that it is a method for precipitating the objective biological molecule by immunochemical technique. The present invention is excellent in that the operation is convenient, cost performance is good and versatility is very high because it can employ the immunochemical technique that can be conducted in most laboratories and examination rooms.

Concretely, in the condition that an immune complex can be formed, a sample, an antibody against the objective biological molecule, and a carrier for precipitation are brought into contact with one another. This causes a reaction of the objective biological molecule with the antibody against the objective biological molecule, and produces an immunoprecipitate in the coexistence of the carrier for precipitation. The carrier for precipitation may be a carrier to which the antibody against the objective biological molecule is bound, or a carrier to which a molecule (for example, anti-IgG antibody, Protein A, Protein G and the like) having immune specific binding capacity to the antibody against the objective biological molecule is bound. In the following, the antibody and the molecule having immune specific bindability are described as "antibody or the like" in some cases. Further, "binding" of the antibody or the like to a carrier is described as "immobilization" or "carrying" of the antibody or the like in some cases.

Further, "reaction" refers to an immunological reaction, and refers to the immunological reaction that is recognizable in the immunological field, and principally an antigen-antibody reaction. According to this reaction, an immune complex containing at least antibody or the like carried on the carrier and the objective biological molecule is obtained as an immunoprecipitate.
molecule having immune specific bindability to the antibody against the objective biological molecule is bound is used as a carrier for precipitation, more concretely, in the condition that a immune complex can be formed, a sample and antibody X against the objective biological molecule are brought into contact with each other, and then a carrier for precipitation carrying secondary antibody Y against the antibody may be brought into contact. According to this manner, the objective biological molecule, the antibody X, and the secondary antibody Y are caused to react, and a complex of the objective biological molecule, the antibody X, and the carrier to which secondary antibody Y is immobilized is obtained as an immunoprecipitate.

Hereinafter, the present invention is further explained with reference to an embodiment that uses a sample, antibody X against the objective biological molecule, and a carrier carrying secondary antibody Y against this antibody.

As the antibody X, any molecule may be used insofar that it is a molecule that immunospecifically binds to the objective biological molecule. The antibody includes a polyclonal antibody, a monoclonal antibody and an antibody prepared by molecular biological technique. Further, the antibody may widely be a substance that immunospecifically binds and an antibody fragment and the like may be used. In any cases, the preparation of the antibody is conducted by a method well known to a person skilled in the art. The present invention is preferable in that the effect of the present invention is satisfactorily obtained even with use of the polyclonal antibody.

The shape of the carrier is not particularly limited. For example, particles (beads) shaped with such as spherical, granular, and crushed may be recited. A carrier with particle (bead) shaped is particularly preferred from the viewpoint of operability, for example, convenience of collecting operation and washing operation of the immunoprecipitate.

The material of the carrier is not particularly limited insofar that it allows binding of the secondary antibody Y. For example, polymer compounds including polysaccharides such as agarose and sepharose, and polymers such as polyacrylamide, polyethylene glycol, polystyrene, polyethylene, polypropylene, polyester, polyacrylonitrile, (meth)acrylate ester polymer, fluorine resin, and metal complex resin may be recited. Further, glass, metal, magnetic substance and the like are also recited. These materials may be used singly or in combination.

The secondary antibody Y to be carried on the carrier is not particularly limited insofar that it is a molecule that binds immunospecifically to the antibody X that immunospecifically binds to the objective biological molecule. As such a molecule, anti-IgG antibody, Protein A, Protein G and the like may be recited.

In the present invention, agarose beads (sepharose), magnetic beads and the like are particularly preferred from the view point of versatility. That is, as these carries, those commercially available from various manufactures may be used. Furthermore, most of these commercially available products are provided in the state that Protein A, Protein G and the like are already bound to the carrier such as sepharose, agarose, magnetic beads or the like.

On the other hand, when an operation of making the carrier carry the secondary antibody Y is conducted, the operation may be appropriately conducted by a person skilled in the art with use of a known carrying method. As the carrying method, for example, physical binding methods, covalent binding methods, ion binding methods and the like are recited, and these methods may be conducted singly or in combination.

While the present invention is explained with referring to the embodiment that uses the sample, the antibody X against the objective biological molecule, and the carrier carrying secondary antibody Y against this antibody, the present invention may be conducted in various other embodiments by a method appropriately selected by a person skilled in the part without being limited to the above embodiment.

The reaction condition of the immunological reaction may be appropriately determined by a person skilled in the art. For example, the reaction time may be about one hour to overnight. The reaction temperature may be for example, about 4 to 10°C. As an example of the standard protocol in the present invention, the reaction condition is about 2 hours at 4°C.

### [3. Washing step of immunoprecipitate]

In the present step, the immunoprecipitate obtained in the above step is appropriately collected and washed with the following washing liquid. The collecting method is appropriately selected by a person skilled in the art.

The washing liquid used in the present step is an aqueous solution containing a charge neutralizing agent. Some of ions derived from salts, molecules and the like coexisting with the immunoprecipitate obtained in the above step would suppress ionization in mass spectrometry when they remain in the later mass spectrometry step. Consequently, by washing the immunoprecipitate with a charge neutralizing agent, it is conceivable that ionization suppression in mass spectrometry can be neutralized by capping.

Further, as the charge neutralizing agent, an agent that keeps pH suited for maintaining the form of the immunoprecipitate is used. The state in which the form of the immunoprecipitate is maintained means the state in which release of the objective biological molecule does not occur as a result of weakening of the antigen-antibody binding capacity in the immunoprecipitate. For this reason, usually, the appropriate pH is not particularly limited insofar that it is not excessively on the acidic region, and preferably 7 or higher, for example, 7 to 9, and further 7 to 8 or 8 to 9.

Further, as the charge neutralizing agent, it is preferred to use an agent that would not interfere with analysis as the background in the later mass spectrometry step.

The charge neutralizing agent used in the present invention produces an ammonium ion in the washing liquid (aqueous solution). The ammonium ion is believed to have a property that it weakly caps charges, for example, of fatty acids or salts. Therefore, as the aqueous solution containing the charge neutralizing agent, diluted ammonia water, aqueous solution of ammonium salt may be recited. A counter anion of ammonium salt may be appropriately determined by a person skilled in the art. Concrete examples of the preferred ammonium salt in the present invention include, for example, ammonium carbonate, ammonium bicarbonate and the like.

The above charge neutralizing agent may be used singly or in combination.

The concentration of the charge neutralizing agent in the washing liquid may be appropriately determined by a person skilled in the art, and for example, 0.1 to 10 mM, for example, about 0.1 mM.

In the present step, the above aqueous solution containing the charge neutralizing agent is used as a washing liquid, and a buffer may be used additionally as a washing liquid. The buffer is not particularly limited, and may be appropriately selected by a person skilled in the art. It is preferred to select the buffer that is less likely to leave salts as much as possible in consideration of influence on the later mass spectrometry step. From the viewpoints, TBS, PBS, HEPES-saline and the like may be recited as a particularly preferred buffer.

It is generally known that binding between antigen and antibody, and protein-protein interaction are optimized in the presence of a certain degree of salt concentration. In this case, washing may be conducted using the buffer in the presence of optimum salts for the purpose of realizing washing without dissociation of the binding between antigen and antibody. The optimum salt and the concentration thereof may be determined appropriately by a person skilled in the art. For example, 150 mM NaCl may be used. In the method of the present invention, since washing is conducted successively using the aqueous solution containing the charge neutralizing agent, there is a merit that charges are efficiently neutralized and that transition to the mass spectrometry step is possible while keeping on the binding with antibody.

Concrete conditions of the amount of the washing liquid and the number of washings may be appropriately determined by a person skilled in the art. Generally and basically, washing operation is conducted using the washing liquid in the amount of about five times of the carrier volume. In the present invention, when the analysis is conducted by conducting washing operation using 100 µL buffer three times, and washing operation using 100 µL aqueous solution containing the charge neutralizing agent once per 5 µL carrier volume, it is confirmed that the analysis is not interfered.

Desirably, washing with the charge neutralizing agent solution is conducted as soon as possible. At the time of exchanging the solution, as an applied example, a spin column having a fine membrane diameter is prepared, and washing operation may be conducted on the filter. Also use of magnetic beads is effective. Consequently, this avoids the risk of sucking out the resin itself, so that improvement in both washing efficiency and collection efficiency can be expected.

### [4. Mass spectrometry step]

The immunoprecipitate washed in the above step is directly subjected to mass spectrometry in a form of a complex without making the objective biological molecule release from the antibody. In the present invention, MALDI (matrix-assisted laser desorption/ionization) based mass spectrometer may be used. The immunoprecipitate may be spotted onto a mass spectrometry sample plate after mixed, for example, with a matrix solution. The matrix may be appropriately determined by a person skilled in the art depending on the objective biological molecule.

When identifying the objective biological molecule, an apparatus capable of conducting at least second order of multi-stage tandem mass spectrometry is used. As an example of such an apparatus, AXIMA-QIT (manufactured by SHIMADZU Corporation) may be recited.

When quantifying the objective biological molecule, the objective biological molecule is measured together with an internal standard, and the quantity may be conducted based on an integration ratio of peaks derived therefrom. The internal standard is not particularly limited, and those appropriately selected by a person skilled in the art may be used in addition to the objective biological molecule. When an immunoprecipitate accompanied with non-specific binding is obtained due to crossing of the antibody used in the concentrating step with other irrelevant molecules, the non-specifically binding molecule may be effectively used as an internal standard besides further addition of the internal standard as described above.

On the other hand, an operation for reducing the non-specifically bound molecule may be conducted appropriately by a person skilled in the art. This operation may be optionally conducted. When this operation is conducted, concretely, a surfactant may be used. However, NP-40 that is generally used in immunoprecipitation is known to suppress mass spectrometry, and those that do not interfere with the later mass spectrometry as much as possible are appropriately selected.

For example, as the surfactant, a nonionic surfactant is preferably used. Concrete examples include, but are not limited to, nonionic surfactants such as n-octyl-β-D-glycoside (n-octyl-β-D-glycopyranoside), n-octyl-β-D-thioglycoside (n-octyl-β-D-thioglycopyranoside), deoxy BIGCHAP, n-dodecyl-β-D-maltoside, n-octyl-β-D-maltoside, sucrose monolaurate, sucrose monocaprate (all is manufactured by DOJINDO Laboratories).

As the nonionic surfactant used in the present invention, a surfactant having a sugar chain as a hydrophilic group is preferred, and a surfactant having a linear alkyl group as a surface-active group is preferred. The length of the linear alkyl chain may be about 8 to 12 carbon number. Wholly, a C8-alkyl glycoside surfactant (that is, a surfactant having a sugar chain as a hydrophilic group and a linear alkyl group having 8 carbon number as a surface-active group) exerts very good effect. Among these, n-octyl-β-D-thioglycoside and n-octyl-β-D-maltoside are particularly preferred.

The surfactant may be, for example, added into the sample prior to the concentrating step by immunoprecipitation or added into the washing liquid during the washing step. When the surfactant is used, the amount used may be appropriately determined by a person skilled in the art. When it is added into the sample prior to the concentrating step by immunoprecipitation, for example, it may be used referring about 0.1 to 2 w/v%, in particular, about 1 w/v%.

### EXAMPLES

In the following, the present invention would be concretely explained by way of Examples, however, the present invention is not limited to these Examples. The mass spectrometry of the immunoprecipitate conducted by the present invention is described as IP-MS (Immunoprecipitation-Mass Spectrometry) in some cases.

### <Example 1: IP-MS basic experiment using amyloid peptide>

A sample liquid (50 µL) containing 10 pmol of amyloid peptide (1-38) (manufactured by Anaspec) in TBS buffer, and a 50 µL antibody liquid containing 0.5 µL amyloid peptide antibody (Anti-amyloid β(1-17), product number NE1003, 6E10, manufactured by Calbiochem) in TBS buffer were prepared, and both liquids were mixed with each other. As for the antibody, the most reliable monoclonal antibody 6E10 clone that is also used in diagnosis of Alzheimer's disease or the like, was used for analysis.

The obtained mixed solution was incubated at 4°C for 2 hours.

Further, the mixed solution was added with Protein G agarose plus (manufactured by PIERCE) (50v/v % slurry in TBS buffer, 5 µL) and incubated at 4°C for 2 hours.

The obtained immunoprecipitate was collected by centrifugal separation, and the collected immunoprecipitate was washed with TBS and 0.1 mM ammonium carbonate aqueous solution. As the washing operation, washing with 100 µL of TBS was conducted three times, and washing with 100 µL of 0.1 mM ammonium carbonate aqueous solution was conducted once.

Equivalent volumes of the washed immunoprecipitate and a matrix solution (solution containing 20 mg/mL of sinapinic acid (manufactured by Wako Pure Chemical Industries) as matrix in 0.1 v/v% trifluoroacetic acid-50 v/v% acetonitrile aqueous solution as solvent) were mixed to prepare a sample for mass spectrometry.

The prepared sample for mass spectrometry was directly spotted on a MALDI target plate.

Using a mass spectrometer (Axima CFR plus, manufactured by SHIMADZU CORPORATION), MALDI-TOF MS was conducted by measuring in linear positive mode.

Fig. 1 shows mass spectrum (horizontal axis: Mass/ Charge, vertical axis: relative ion intensity (%Int.)) obtained by the above operation. In Fig. 1, mass spectrum (IP) from the immunoprecipitate is shown together with mass spectrum (Aβ) from amyloid (1-38) control, mass spectrum (Before IP) from a solution just after mixing with antibody, and mass spectrum (IP sup.) from the supernatant after immunoprecipitation.

As shown in Fig. 1, in the data (Aβ) of control and the data (Before IP) from a solution just after mixing with antibody, a signal derived from amyloid peptide is observed, while in the data (IP sup.) from the supernatant after immunoprecipitation, the signal is not detected. Further, in the data (IP) from the immunoprecipitate, a signal equivalent to that in the case of the data (Aβ) of control is detected. Consequently, it is demonstrated that the experimental system of the present invention works well.

### <Example 2: IP-MS sensitivity test>

An amount of amyloid peptide (1-38) in the sample in Example 1 was varied to 100 pmol, 1 pmol, 100 fmol, 10 fmol and 1 fmol, and samples each having these amounts of amyloid peptide(1-38) were subjected to the same operation as in Example 1. The obtained mass spectrums are shown in Fig. 2 together with the mass spectrum (amount of amyloid peptide (1-38) in sample: 10 pmol) obtained in Example 1. As shown in Fig. 2, a good signal could be obtained even when the amount of amyloid peptide (1-38) in the sample was 1 fmol.

### <Example 3: Sensitivity test in serum of IP-MS>

The same operation as in Example 1 and Example 2 was conducted except that serum from which an Ig molecule was removed was used in place of the buffer. That is, samples each containing 100 pmol, 10 pmol, 1 pmol, 100 fmol, 10 fmol and 1 fmol amyloid peptide (1-38) in the serum from which the Ig molecule was removed were prepared, and IP-MS was conducted on each sample.

Removal of the Ig molecule in serum is very important in immunoprecipitation. This is because when pull-down is conducted with Protein G without removing the Ig molecules, efficiency of the immunoprecipitation decreases due to binding of existent Ig, and necessity of large excess of antibody and Protein G agarose resin, so that there is a fear that sensitivity of the analysis system may be significantly deteriorated.

The serum from which the Ig molecule was removed was prepared by diluting 50 µL of serum to five-fold with TBS, adding with Protein G agarose plus (manufactured by PIERCE), shaking it at 4°C for 2 hours, and then the conducting centrifugal separation to collect the supernatant.

The obtained mass spectrum is shown in Fig. 3. As shown in Fig. 3, a good signal could be obtained even when the amount of amyloid peptide (1-38) in the serum sample was 1 fmol. The possibility to detect a molecule in an amount of 1 fmol in the serum sample means that a molecule of several ng/mL in terms of concentration is detectable. Therefore, it is considered that the accuracy is enough to detect in the concentration that is necessary to have significance as a biomarker.

Also as shown in Fig. 3, those that seem to be molecules specifically crossing and nonspecifically binding to an amyloid peptide antibody ("Antibody-crossed non-specific binding component") are detected. As an approach for such a non-specific binding, a technique of reducing the non-specific binding, and a technique of utilizing the non-specific binding are possible.

The technique of reducing non-specific binding is not particularly limited, and may be appropriately conducted by a person skilled in the art. In a usual immunoprecipitation, NP-40 is used. However, the NP-40 tends to strongly suppress mass spectrometry and exert a great inhibitory effect on an analyzer. From this viewpoint, the present inventors have examined a plurality of surfactants, and have found that a nonionic surfactant having a sugar group as a hydrophilic group and a linear alkyl group having 8 carbon number as a surface-active group is particularly very useful (Example 4 and Example 7 below). Concretely, for example, in the above Examples, an operation of adding, for example, about 0.1 to 2w/v%, particularly about 1 w/v% of surfactant into serum before IP-MS (namely, before concentrating step by immunoprecipitation) may additionally be conducted.

On the other hand, as a technique of utilizing the non-specific binding, the antibody-crossed non-specific binding component may be used as an internal standard. This is based on the fact that peak intensity of the antibody-crossed non-specific binding component changes little regardless of the wide range of variation in concentration of amyloid peptide ranging from 100 pmol to 1 fmol as shown in Fig. 3.

### <Example 4: Choice of surfactant for reducing non-specific binding in IP-MS>

In the present Example, an experiment was conducted while adding a surfactant into the serum from which the Ig molecule was removed as used in Example 3. As the surfactant, n-octyl-β-D-glycoside (n-octyl-β-D-glycopyranoside) and n-octyl-β-D-thioglycoside (n-octyl-β-D-thioglycopyranoside) were examined, respectively. Concretely, to the 50 µL of serum from which the Ig molecule was removed, the surfactant was added so as to have a final concentration of 1 w/v%, and the mixture was voltexed at room temperature for 15 minutes. The same operation was conducted as in Example 3 except that the surfactant-containing serum obtained in this manner was used in place of the serum from which the Ig molecule was removed in Example 3. The amount of amyloid peptide (1-38) in the sample was 1 pmol.

The obtained mass spectrum is shown in Fig. 4. The strongest signal intensity of amyloid peptide was obtained when 1 w/v% n-octyl-β-D-thioglycoside was added. In comparison with spectrum (None) of the control in which the surfactant is not added into the serum, significant improvement was observed in signal intensity of amyloid peptide, and in signal/noise ratio.

### <Example 5: Measurement of amyloid peptide mixture by IP-MS>

In clinical diagnosis, the abundance ratio of amyloid peptide, in particular, (1-40)/(1-42) ratio, is very important.

In the present Example, a mixture containing a trace amount of amyloid peptide was subjected to IP-MS.

Concretely, mass spectrum was obtained by the same operation as in Example 1 except that 1 pmol of amyloid peptide(1-38) in the sample liquid in Example 4 was replaced by a mixture of each 500 fmol of amyloid (1-38), amyloid (1-40) and amyloid (1-42) and a solution condition containing 1% n-octyl-β-D-thioglycoside was employed.

Fig. 5 shows (a) mass spectrum of amyloid peptide mixture (Mixture), together with (b) mass spectrum of 500 fmol amyloid peptide(1-42), (c) mass spectrum of 500 fmol amyloid peptide(1-40), and (d) mass spectrum of 500 fmol amyloid peptide(1-38) (horizontal axis: Mass/Charge, vertical axis: relative ion intensity (%Int.)).

The result of Fig. 5 demonstrates that the above mixture could be measured simultaneously by the identical antibody with use of IP-MS as a platform. Here, since ionization efficiency of each peptide is inherent, peak intensity thereof differs from each other. By appropriately correcting the peak intensity by a method known to a person skilled in the art (for example, correction by using an external standard as in Figs. 5(b) to (d)), the abundance ratio can be quantified.

As already described, amyloid (1-40) and amyloid (1-42) can be detected by the identical antibody even with a generally used ELISA method, however, the ELISA method fails to distinguish from each other. In other words, when the ELISA method is used, antibodies specific for each of amyloid (1-40) and amyloid (1-42) are required to distinguish from each other.

The IP-MS method of the present invention is a very innovative method that makes it possible to detect a peptide mixture in a trace amount scale as described above as an ion peak having mass difference by mass spectrometry for the first time. Also the IP-MS method of the present invention makes it possible to find a mixing ratio of proteins in a trace amount which is very important in clinical diagnosis, such as the amyloid (1-40)/(1-42) ratio, with a very simple method.

### <Example 6: Measurement of amyloid peptide in plasma and in cell lysate by IP-MS>

In the present Example, amyloid peptides in serum, in plasma and in cell lysate which is a treated liquid obtained by culture were measured.

As the serum, serum identical to that in Example 3 was used.

As the plasma, plasma purchased from COSMO BIO (catalogue No. 12250110) was used.

As the cell lysate, cell lysate obtained in the following manner was used. HEK293T cells were cultured for 3 days in DMEM (4.5 w/v% high glucose) + 10 v/v% FBS on 15 cm dish. The cultured cells were washed with a phosphate buffer solution, and then collected. The collected culture cells were suspended in 1 mL of Tris buffer (containing protease inhibitor and 1 w/v% n-octyl-β-D-thioglycoside), and crushed by centrifugating at 5000 rpm for 120 seconds in a cooling bath by using zirconia beads of 1 mm in diameter. Thereafter, centrifugation at 20000g was conducted at 4°C for 30 minutes, and the supernatant was obtained as a cell lysate.

For each of 50 µL of the above serum, 50 µL of the above plasma, and 50 µL of the above cell lysate, the following steps were conducted. Diluting in 200 µL of Tris buffer (containing 0.5 w/v% n-octyl-β-D-thioglycoside), and the obtained diluted liquid was added with Protein G agarose beads (manufactured by PIERCE), and the mixture was stirred at 4°C for 2 hours. Thereafter, the supernatant was collected by centrifugation.

500 fmol of amyloid peptide and 0.5 µL of antibody were added to the collected supernatant, and the mixture was stirred gently at 4°C for 2 hours. After stirring, 5 µL of Protein G agarose beads were further added thereto, and the mixture was stirred at 4°C for 2 hours, to allow amyloid peptide immunoprecipitate. The beads (immunoprecipitate) were washed with Tris buffer and 0.1 mM ammonium bicarbonate solution, and the supernatant was removed. The remaining beads (immunoprecipitate) were mixed with 5 µL of 20mg/mL sinapinic acid solution which is identical to the matrix solution used in Example 1, and a beads suspension was obtained. The beads suspension was directly spotted onto a MALDI target plate, and subjected to mass spectrometry after drying.

Fig. 6 shows (a) mass spectrum of amyloid in cell lysate, (b) mass spectrum of amyloid in plasma, and (c) mass spectrum of amyloid in serum (horizontal axis: Mass/Charge, vertical axis: relative ion intensity (%Int.)). As shown in Fig. 6, amyloid can be detected with good sensitivity in any of the serum, plasma and cell lysate.

### <Example 7: Screening of surfactant for reducing non-specific binding in IP-MS>

In the present Example, surfactants other than the surfactant used in Example 4 were also evaluated.

Concretely, as the surfactant, n-octyl-β-D-thioglycoside (OTG), deoxy BIGCHAP (BIGCHAP), n-dodecyl-β-D-maltoside (DDM), n-octyl-β-D-maltoside (OM), sucrose monolaurate (SML), and sucrose monocaprate (SMC) (all is manufactured by DOJINDO Laboratories) were used, respectively. The same operation as in Example 4 was conducted except that the surfactant in Example 4 was replaced by the respective surfactants, and mass spectrums were obtained.

The obtained mass spectrums are shown in Fig. 7 (horizontal axis: Mass/Charge, vertical axis: relative ion intensity (%Int.)). Concretely, the mass spectrums are obtained by conducting IP-MS when using (a): sucrose monocaprate (SMC), (b): sucrose monolaurate(SML), (c): n-octyl-β-D-Maltoside (OM), (d): n-dodecyl-β-D-maltoside (DDM), (e): deoxy BIGCHAP (BIGCHAP), and (f): n-octyl-β-D-thioglycoside (OTG)), respectively, as a surfactant. Each spectrum is shown divisionally in the range of m/z 300 to 2000 and in the range of m/z 2000 to 10000. Relative intensity of peak is appropriately optimized for representation in order to see a peak of surfactant in the range of m/z 300 to 2000, and a peak of amyloid peptide in the range of m/z 2000 to 10000.

As shown in Fig. 7, wholly, the nonionic surfactants having a sugar group as a hydrophilic group and a linear alkyl group having 8 carbon number as a surface-active group exhibited very good results. It was demonstrated that among these, for example, OTG could be preferably selected as a surfactant having a little non-specific binding peak, and OM could be selected as a surfactant that is able to collect an objective substance efficiently, or makes it possible to conduct IP-MS in a mild condition despite large non-specific binding.

While concrete forms within the scope of the present invention have been described in the above Examples, the present invention may be practiced in various other forms without limited to these. Accordingly, the above Examples are given merely for exemplification in every terms, and should not be interpreted in a limitative manner. Further, any modifications within equivalents of claims are included in the scope of the present invention.

## Claims

1. Mass spectrometry of a biological sample using immunoprecipitation,
comprising the steps of:
preparing a sample containing an objective biological molecule selected from the group consisting of a peptide and a protein;
concentrating the objective biological molecule in the sample by immunoprecipitation using an antibody-bound carrier or a carrier to which a molecule capable of specifically binding to an antibody is bound, to obtain an immune complex as an immunoprecipitate containing at least said antibody or said molecule carried on the carrier and the objective biological molecule;
washing the immunoprecipitate of the objective biological molecule by using an aqueous solution containing a charge neutralizing agent as a washing liquid; and
detecting the objective biological molecule by directly subjecting the washed immunoprecipitate to mass spectrometry.

2. The mass spectrometry of a biological sample using immunoprecipitation according to claim 1, wherein the sample containing an objective biological molecule is contained in a body fluid selected from the group consisting of blood, urine, cerebrospinal fluid and body secretion fluid.

3. The mass spectrometry of a biological sample using immunoprecipitation according to claim 1, wherein the sample containing an objective biological molecule is a clinical sample.

4. The mass spectrometry of a biological sample using immunoprecipitation according to claim 1, wherein the biological molecule is selected from the group consisting of a biomarker, biological molecule of a biomarker candidate, and a fragment derived from the biomarker or the biological molecule of a biomarker candidate by splicing.

5. The mass spectrometry of a biological sample using immunoprecipitation according to claim 4, wherein the biomarker and the biological molecule of a biomarker candidate are responsive to a disease selected from the group consisting of a cancer, a brain disease, an immune disease, a liver disease, a renal disease and an eye disease.

6. The mass spectrometry of a biological sample using immunoprecipitation according to claim 1, wherein the carrier is selected from the group consisting of agarose, sepharose, polyacrylamide, polyethylene glycol, polystyrene, polyethylene, polypropylene, polyester, polyacrylonitrile, (meth)acrylate ester polymer, fluorine resin, metal complex resin, glass, metal and magnetic substance.

7. The mass spectrometry of a biological sample using immunoprecipitation according to claim 1, wherein the aqueous solution containing the charge neutralizing agent is selected from the group consisting of ammonia water and aqueous solution of ammonium salt.

8. The mass spectrometry of a biological sample using immunoprecipitation according to any one of claims 1 to 7, wherein in the mass spectrometry, multi-stage tandem mass spectrometry is conducted.

9. The mass spectrometry of a biological sample using immunoprecipitation according to claim 1, wherein in the mass spectrometry, the biological molecule is quantified by measuring the washed immunoprecipitate in the presence of an internal standard substance.

## Patentansprüche

1. Massenspektrometrie einer biologischen Probe unter Anwendung von Immunpräzipitation, umfassend die Schritte:
Herstellen einer Probe, welche ein biologisches Zielmolekül enthält, das aus der Gruppe bestehend aus einem Peptid und einem Protein ausgewählt ist;
Konzentrieren des biologischen Zielmoleküls in der Probe durch Immunpräzipitation unter Verwendung eines Antikörper-gebundenen Trägers oder eines Trägers, an den ein Molekül gebunden ist, welches spezifisch an einen Antikörper binden kann, zum Erhalt eines Immunkomplexes als Immunpräzipitat, welches wenigstens den Antikörper oder das auf dem Träger getragene Molekül und das biologische Zielmolekül enthält;
Waschen des Immunpräzipitats des biologischen Zielmoleküls unter Verwendung einer wässrigen Lösung, welche ein Ladungsneutralisierendes Agens enthält, als Waschflüssigkelt; und
Nachweis des biologischen Zielmoleküls durch direkte Durchführung einer Massenspektrometrie an dem gewaschenen Immunpräzipitat.

2. Massenspektrometrie einer biologischen Probe unter Anwendung von Immunpräzipitation gemäß Anspruch 1, wobei die ein biologisches Zielmolekül enthaltende Probe in einer Körperflüssigkeit enthalten ist, die aus Blut, Urin, Zerebrospinalflüssigkeit und Sekretionsflüssigkeit des Körpers ausgewählt ist.

3. Massenspektrometrie einer biologischen Probe unter Anwendung von Immunpräzipitation gemäß Anspruch 1, wobei die ein biologisches Zielmolekül enthaltende Probe eine klinische Probe ist.

4. Massenspektrometrie einer biologischen Probe unter Anwendung von Immunpräzipitation gemäß Anspruch 1, wobei das biologische Molekül aus der Gruppe bestehend aus einem Biomarker, einem biologischen Molekül eines Biomarker-Kandidaten und einem Fragment, welches von dem Biomarker oder dem biologischen Molekül eines Biomarker-Kandidaten durch Spleißen abgeleitet ist, ausgewählt ist.

5. Massenspektrometrie einer biologisches Probe unter Anwendung von Immunpräzipitation gemäß Anspruch 4, wobei der Biomarker und das biologische Molekül eines Biomarker-Kandidaten auf eine Krankheit ansprechen, die aus der Gruppe bestehend aus einer Krebserkrankung, einer Gehirnerkrankung, einer Immunerkrankung, einer Lebererkrankung, einer Nierenerkrankung und einer Augenerkrankung ausgewählt ist.

6. Massenspektrometrie einer biologischen Probe unter Anwendung von Immunpräzipitation gemäß Anspruch 1, wobei der Träger aus der Gruppe bestehend aus Agarose, Sepharose, Polyacrylamid, Polyethylenglykol, Polystyrol, Polyethylen, Polypropylen, Polyester, Polyacrylnitril, (Meth)acrylatester-Polymer, Fluorharz, Metallkomplex-Harz, Glas, Metall und einer magnetischen Substanz ausgewählt ist.

7. Massenspektrometrie einer biologischen Probe unter Anwendung von Immunpräzipitation gemäß Anspruch 1, wobei die wässrige Lösung, welche das Ladungs-neutralisierende Agens enthält, aus der Gruppe bestehend aus Ammoniakwasser und einer wässrigen Lösung eines Ammoniumsalzes ausgewählt ist.

8. Massenspektrometrie einer biologischen Probe unter Anwendung von Immunpräzipitation gemäß irgendeinem der Ansprüche 1 bis 7, wobei in der Massenspektrometrie eine mehrstufige (multi-stage) Tandem-Massenspektrometrie durchgeführt wird.

9. Massenspektrometrle einer biologischen Probe unter Anwendung von Immunpräzipitation gemäß Anspruch 1, wobei in der Massenspektrometrie das biologische Molekül durch Messen des gewaschenen Immunpräzipltats In Gegenwart einer Internen Standardsubstanz quantifiziert wird.

## Revendications

1. Spectrométrie de masse d'un échantillon biologique utilisant l'immunoprécipitation,
comprenant les étapes de :
préparation d'un échantillon contenant une molécule biologique objective sélectionnée dans le groupe constitué d'un peptide et d'une protéine ;
concentration de la molécule biologique objective dans l'échantillon par immunoprécipitation en utilisant un vecteur lié à un anticorps ou un vecteur auquel est liée une molécule capable de spécifiquement se lier à un anticorps, pour obtenir un complexe immunitaire sous la forme d'un immunoprécipité contenant au moins ledit anticorps ou ladite molécule portée sur le vecteur et la molécule biologique objective ;
laver l'immunoprécipité de la molécule biologique objective en utilisant une solution aqueuse contenant un agent de neutralisation des charges en tant que liquide de lavage ; et
détecter la molécule biologique objective en soumettant directement l'immunoprécipité lavé à la spectrométrie de masse.

2. Spectrométrie de masse d'un échantillon biologique utilisant l'immunoprécipitation selon la revendication 1, dans laquelle l'échantillon contenant une molécule biologique objective est contenu dans un fluide corporel sélectionné dans le groupe consistant en le sang, l'urine, le fluide céphalorachidien et un fluide de sécrétion corporelle.

3. Spectrométrie de masse d'un échantillon biologique utilisant l'immunoprécipitation selon la revendication 1, dans laquelle l'échantillon contenant une molécule biologique objective est un échantillon clinique.

4. Spectrométrie de masse d'un échantillon biologique utilisant l'immunoprécipitation selon la revendication 1, dans laquelle la molécule biologique est sélectionnée dans le groupe consistant en un biomarqueur, une molécule biologique d'un candidat biomarqueur, et d'un fragment dérivé du biomarqueur ou de la molécule biologique d'un candidat biomarqueur par épissage.

5. Spectrométrie de masse d'un échantillon biologique utilisant l'immunoprécipitation selon la revendication 4, dans laquelle le biomarqueur et la molécule biologique d'un candidat biomarqueur sont réactifs à une maladie sélectionnée dans le groupe consistant en un cancer, une maladie cérébrale, d'une maladie immunitaire, d'une maladie hépatique, d'une maladie rénale et d'une maladie oculaire.

6. Spectrométrie de masse d'un échantillon biologique utilisant l'immunoprécipitation selon la revendication 1, dans laquelle le vecteur est sélectionné dans le groupe consistant en de l'agarose, la sépharose, un polyacrylamide, un polyéthylène glycol, un polystyrène, un polyéthylène, un polypropylène, un polyester, un polyacrylonitrile, un polymère d'ester de (méth)acrylate, une résine fluor, une résine à base de complexe métallique, du verre, un métal et une substance magnétique.

7. Spectrométrie de masse d'un échantillon biologique utilisant l'immunoprécipitation selon la revendication 1, dans laquelle la solution aqueuse contenant l'agent de neutralisation des charges est sélectionnée dans le groupe constitué de l'eau ammoniacale et d'une solution aqueuse de sel d'ammonium.

8. Spectrométrie de masse d'un échantillon biologique utilisant l'immunoprécipitation selon l'une quelconque des revendications 1 à 7, dans laquelle dans la Spectrométrie de masse, une spectrométrie de masse en tandem à étapes multiples est réalisée.

9. Spectrométrie de masse d'un échantillon biologique utilisant l'immunoprécipitation selon la revendication 1, dans laquelle dans la spectrométrie de masse, la molécule biologique est quantifiée par mesure de l'immunoprécipité lavé en présence d'une substance étalon interne.
